# EUROPÄISCHE PATENTSCHRIFT

(11) **EP 2 248 144 B1**
(45) Veröffentlichungstag und Bekanntmachung des Hinweises auf die Patenterteilung: **05.03.2014**
(21) Anmeldenummer: 09713707.9
(22) Anmeldetag: 23.01.2009
(51) Int. Cl.: H01J 27/18

(54) **PARTIKELTHERAPIEANLAGE**
PARTICLE THERAPY SYSTEM
SYSTÈME DE THÉRAPIE PAR PARTICULES

(30) Priorität: 25.02.2008 DE 102008011015
(43) Veröffentlichungstag der Anmeldung: 10.11.2010
(73) Patentinhaber: Siemens Aktiengesellschaft, 80333 München (DE)
(72) Erfinder: TANKE, Eugene, East Lansing, 48823 MI (US)
(86) Internationale Anmeldenummer: PCT/EP2009/050736
(87) Internationale Veröffentlichungsnummer: WO 2009/106389

(56) Entgegenhaltungen:
- EP-A- 0 967 628
- EP-A- 0 986 070
- DE-A1- 19 933 762
- LAMY T ET AL: "Status of charge breeding with electron cyclotron resonance ion sources (invited)" REVIEW OF SCIENTIFIC INSTRUMENTS, AIP, MELVILLE, NY, US, Bd. 77, Nr. 3, 24. Februar 2006 (2006-02-24), Seiten 3B101-03B101, XP012092900 ISSN: 0034-6748
- ZAVODSZKY ET AL: "Trends in the production of highly charged ions" NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 261, Nr. 1-2, 19. Juli 2007 (2007-07-19), Seiten 1-4, XP022151851 ISSN: 0168-583X

## Beschreibung

Die Erfindung betrifft eine Partikeltherapieanlage wie sie insbesondere zur Behandlung von Tumorerkrankungen eingesetzt wird.

Die Partikeltherapie ist ein etabliertes Verfahren zur Behandlung von Gewebe, insbesondere von Tumorerkrankungen. Hierbei werden üblicherweise geladene Partikel auf hohe Energien beschleunigt, zu einem Partikelstrahl geformt und über ein Hochenergiestrahl-Transportsystem zu einem oder mehreren Bestrahlungsräumen geführt. Die Partikeltherapie weist dabei den Vorteil auf, dass der Partikelstrahl mit dem zu bestrahlenden Gewebe in einem relativ engen, umschriebenen Bereich wechselwirkt, so dass umliegendes, zu schonendes Gewebe effektiv geschont werden kann. Als Partikel werden in der Partikeltherapie neben Protonen, Heliumionen oder Pionen auch andere leichte Ionen, d.h. Ionen mit einer Kernladungszahl kleiner oder gleich 10 wie beispielsweise Kohlenstoffionen oder Sauerstoffionen, zur Bestrahlung eingesetzt.

Derartige Ionen können beispielsweise durch eine so genannte ECR-Ionenquelle (ECR für engl.: "electron cyclotron resonance) bereitgestellt werden. In einer ECR-Ionenquelle befindet sich Plasma, das in einem Magnetfeld eingeschlossen ist. Das Magnetfeld umfasst eine axiale Komponente und eine radiale Komponente und ist derart beschaffen, dass Plasmaelektronen, die sich durch die Lorentz-Kraft auf Spiralbahnen um die magnetischen Feldlinien bewegen, durch Einstrahlen von Mikrowellenstrahlung auf hohe Energien beschleunigt werden können. Die Mikrowellenstrahlung ist dabei auf die Magnetfelder im Plasma abgestimmt, um resonant auf Plasmaelektronen zu wirken. Die Plasmaelektronen, geheizt durch die Mikrowellenstrahlung, ionisieren ihrerseits wiederum Atome und/oder Ionen, die weiter ionisiert werden. Die im Plasma entstehenden Ionen können aus der ECR-Ionenquelle mit einer Hochspannung extrahiert werden. Während der Einstrahlung der Mikrowellenstrahlung tritt hierdurch ein Ionenstrom aus der ECR-Ionenquelle aus.

Das Dokument EP-A-0 986 070 A1 offenbart eine Partikeltherapieanlage mit einer ECR-Ionenquelle im DC-Modus betrieben zur Erzeugung von geladenen Ionen, die in einer der ECR-Ionenquelle nachfolgenden Beschleunigereinheit auf eine zur Bestrahlung notwendige Energie beschleunigt werden, wobei die Magnetfelder der ECR-Ionenquelle auf einen Betrieb der ECR-Ionenquelle für leichte Ionen abgestimmt sind.

Beim Betrieb einer ECR-Ionenquelle hat sich gezeigt, dass der austretende Ionenstrom oftmals im Laufe der Zeit abnimmt. Dies kann im Fall von Kohlenstoffionen z.B. auf Ablagerungen von Atomen des Plasmagases zurückgeführt werden, die sich an den Wänden der ECR-Ionenquelle im Laufe des Betriebs ablagern. Um dennoch einen ausreichenden Ionenstrom und damit einen sicheren Betrieb der Partikeltherapieanlage zu ermöglichen, muss die ECR-Ionenquelle daher entsprechend dimensioniert oder entsprechend oft gewartet werden, was insgesamt zu höheren Kosten und größerem Aufwand führt.

In dem Artikel "Performance of the ECR Ion Source of CERN's Heavy Ion Injector" M.P. Bougarel et al., PS/HI Note 95-12, presented at the 12th International Workshop on ECR Ion Sources, April 25-27, 1995, RIKEN, Japan, wird unter anderem der so genannte Afterglow-Modus einer ECR-Ionenquelle beschrieben. Die ECR-Ionenquelle wird dabei gepulst betrieben, d.h. die Mikrowellenstrahlung wird nicht kontinuierlich, sondern gepulst angewendet. Dabei wurde beobachtet, dass nach Abschaltung eines Mikrowellen-Strahlungspulses (d.h. in der Nachglüh-Phase, engl. "afterglow") ein kurzer Ionenpuls mit hoher Stromstärke aus der ECR-Ionenquelle emittiert wird.

In dem Artikel "HIMAC and Medical Accelerator Projects in Japan", S. Yamada et al., Mar 1998, Proceedings of 1st Asian Particle Accelerator Conference (APAC 98), Tsukuba, Japan, 23-27 Mar 1998, pp 885, ist beschrieben, dass die Ionenquelle der Anlage mit Krypton-Ionen bzw. mit Eisen-Ionen im so genannten Afterglow-Modus betrieben wurde.

Es ist die Aufgabe der Erfindung, eine Partikeltherapieanlage mit einer Ionenquelle bereitzustellen, bei der hohe Strahlintensitäten bei gleichzeitig geringer Dimensionierung möglich sind.

Die Aufgabe wird erfindungsgemäß gelöst durch eine Partikeltherapieanlage nach Anspruch 1. Vorteilhafte Weiterbildungen finden sich in den Merkmalen der abhängigen Ansprüche.

Die erfindungsgemäße Partikeltherapieanlage umfasst eine ECR-Ionenquelle zur Erzeugung von geladenen Ionen, die in einer der ECR-Ionenquelle nachfolgenden Beschleunigereinheit auf eine zur Bestrahlung notwendige Energie beschleunigt werden. Die Magnetfelder der ECR-Ionenquelle sind dabei auf einen Betrieb der ECR-Ionenquelle mit leichten Ionen, d.h. mit Ionen, deren Kernladungszahl kleiner oder gleich 10 ist, abgestimmt. Die ECR-Ionenquelle wird dabei im Afterglow-Modus betrieben, bei dem nach Abschalten eines Mikrowellen-Resonanzpulses ein Afterglow-Strahlen-Puls aus der ECR-Ionenquelle austritt, der einen höhere Stromstärke aufweist verglichen mit der Stromstärke des Ionenstroms, der aus der ECR-Ionenquelle während der Anwendung des Mikrowellen-Resonanzpulses austritt.

Dabei liegt die Idee zu Grunde, die hohe Stromstärke von Ionen aus der ECR-Ionenquelle, die während eines Afterglow-Strahlen-Pulses auftritt, zu nutzen. Auf diese Weise kann die ECR-Ionenquelle insgesamt kleiner und damit kostengünstiger dimensioniert werden, da die erforderliche Stromstärke durch die höhere Stromstärke des Afterglow-Strahlen-Pulses bereitgestellt wird.

Die Magnetfelder der ECR-Ionenquelle sind dabei so dimensioniert, dass die ECR-Ionenquelle auch mit leichten Ionen betrieben werden kann. Insbesondere kann eine derartige ECR-Ionenquelle mit Kohlenstoffionen und/oder Sauerstoffionen betrieben werden. Insbesondere bei Kohlenstoffionen kann es sich als vorteilhaft erweisen, die ECR-Ionenquelle gepulst im Afterglow-Modus zu betreiben, da so weniger Ablagerungen an den Wänden der ECR-Ionenquelle auftreten als bei einem kontinuierlichen Betriebsmodus der ECR-Ionenquelle.

Die nachfolgende Beschleunigereinheit umfasst üblicherweise einen Linearbeschleuniger, der in einer vorteilhaften Weise derart betrieben wird, dass sein Timing auf das Timing der ECR-Ionenquelle abgestimmt ist, und zwar derart, dass zumindest ein Teil des Ionenstroms, der während des Afterglow-Strahlen-Pulses aus der ECR-Ionenquelle austritt, durch die nachfolgende Beschleunigereinheit beschleunigt wird.

Insbesondere können lediglich diejenigen Ionen zur weiteren Beschleunigung verwendet werden, die durch den Afterglow-Strahlen-Puls bereitgestellt werden. Dies ist deshalb möglich, da die Dauer eines Afterglow-Strahlen-Pulses üblicherweise lang genug ist, so dass die durch den Afterglow-Strahlen-Puls bereitgestellten Ionen bereits ausreichen, um die nachfolgende Beschleunigereinheit zu speisen.

Im Gegensatz zu ECR-Ionenquellen, die kontinuierlich betrieben werden, ist damit kein spezielles, Strahlpuls formendes Element notwendig, wie beispielsweise ein so genannter Chopper, der zwischen einer ECR-Ionenquelle und einem nachfolgenden Linearbeschleuniger eingefügt ist, falls die ECR-Ionenquelle im kontinuierlichen Betriebsmodus betrieben wird. Der nachfolgende Linearbeschleuniger kann beispielsweise einen so genannten RFQ-Beschleuniger umfassen (RFQ für engl.: "Radio Frequency Quadrupole").

Die ECR-Ionenquelle weist üblicherweise ein Magnetsystem auf, das in der ECR-Ionenquelle ein axiales Magnetfeld erzeugt. In einer vorteilhaften Ausführungsvariante umfasst dieses Magnetsystem zumindest einen Permanentmagneten und zusätzlich zum Permanentmagneten zumindest einen variabel einstellbaren Elektromagneten.

Zusätzlich zu dem Magnetsystem für das axiale Magnetfeld weist die ECR-Ionenquelle ein Magnetsystem für das radiale Magnetfeld auf. Beispielsweise kann hier ein Hexapol-Magnetfeld erzeugt werden. Zusätzlich oder alternativ zu vorgenannter Ausführungsvariante umfasst das Magnetsystem für das radiale Magnetfeld ebenfalls zumindest einen Permanentmagneten und zusätzlich zu dem Permanentmagneten zumindest einen variabel einstellbaren Elektromagneten.

Dies hat den Vorteil, dass über den oder die variabel einstellbaren Elektromagneten das axialen Magnetfeld bzw. das radiale Magnetfeld der ECR-Ionenquelle auf einfache und schnelle Art und Weise justiert und angepasst werden kann. Wenn beispielsweise eine ECR-Ionenquelle mit Kohlenstoffionen und Sauerstoffionen betrieben werden soll, kann über den oder die variabel einstellbaren Elektromagneten das axiale Magnetfeld bzw. das radiale Magnetfeld schnell und einfach der verwendeten Ionensorte angepasst werden. Ebenso eröffnet sich so die Möglichkeit, eine Feineinstellung der ECR-Ionenquelle einfach zu realisieren. Im Vergleich zu ECR-Ionenquellen, deren axiales Magnetfeld bzw. deren radiales Magnetfeld vollständig durch Elektromagnete erzeugt wird, können der oder die in Kombination mit einem oder mehreren Permanentmagneten verwendeten Elektromagnete deutlich kostengünstiger und Energie sparender ausgebildet werden.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen Überblick über eine Partikeltherapieanlage,
- Fig. 2: einen Längsschnitt durch eine ECR-Ionenquelle,
- Fig. 3: einen schematischen Überblick über den ersten Abschnitt des Beschleunigers bei einer Partikeltherapieanlage, und
- Fig. 4: ein Diagramm zur Darstellung des Timings der ECR-Ionenquelle und der nachgeschalteten linearen Beschleunigereinheit.

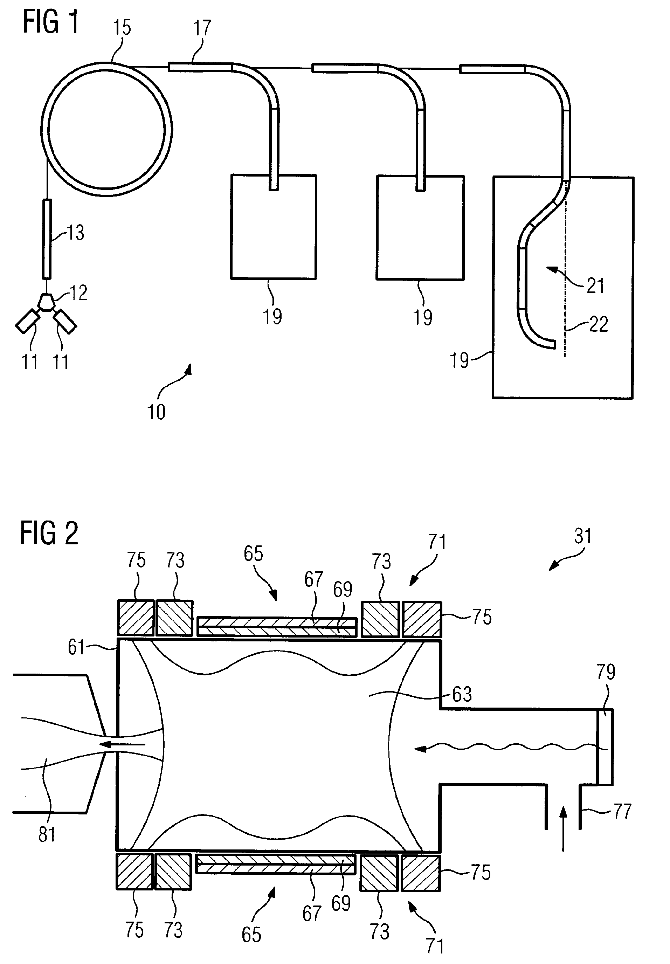

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Io-nensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Aufbau einer Partikeltherapieanlage 10 ist im Stand der Technik bekannt und typisch für viele Partikeltherapieanlagen, kann aber auch hiervon abweichen.

Die anhand der nachfolgenden Figuren beschriebenen Ausführungsformen der Erfindung können in einer derartigen Partikeltherapieanlage 10 Anwendung finden.

Fig. 2 zeigt einen Längsschnitt durch eine ECR-Ionenquelle 31.

Die ECR-Ionenquelle 31 umfasst eine Kammer 61, in der sich das zu heizende Plasma 63 befindet. Um diese Kammer 61 sind mehrere Magnetsysteme angeordnet. Ein erstes Magnetsystem 65 erzeugt ein radiales Magnetfeld. Das erste Magnetsystem 65 ist hier - schematisch angedeutet - als ein Magnetsystem dargestellt, das sowohl zumindest einen Permanentmagnet 69 als auch einen Elektromagnet 67 für das radiale Magnetfeld umfasst. Der Permanentmagnet 69 erzeugt dabei eine statische Grundkomponente des radialen Magnetfeldes, der Elektromagnet 67 überlagert dieser statischen Grundkomponente weitere radiale Komponenten des Magnetfeldes.

Ein zweites Magnetsystem 71 erzeugt ein axiales Magnetfeld. Das zweite Magnetsystem 71 umfasst zumindest einen Permanentmagnet 73 sowie zumindest einen Elektromagnet 75 - auch hier lediglich schematisch angedeutet. Der Permanentmagnet 73 erzeugt dabei eine statische Grundkomponente des axialen Magnetfeldes, der Elektromagnet 75 überlagert dieser statischen Grundkomponente weitere axiale Magnetfeldkomponenten.

Der Elektromagnet 67 für das radiale Magnetfeld und/oder der Elektromagnet 75 für das axiale Magnetfeld können über eine Steuereinheit angesteuert und eingestellt werden. Hierdurch ist es möglich, am axialen Magnetfeld bzw. am radialen Magnetfeld auf einfache Weise variable Einstellungen, insbesondere Feineinstellungen, vorzunehmen. Ebenso lässt sich so das Magnetfeld leicht anpassen, wenn die ECR-Ionenquelle 31 mit einer anderen Ionensorte betrieben werden soll. Da zumindest ein Teil der axialen Magnetfeldkomponente bzw. der radialen Magnetfeldkomponente durch die Permanentmagnete 69, 73 bereitgestellt wird, können die Elektromagnete 67, 75 entsprechend kleiner dimensioniert und dadurch günstiger betrieben werden.

Auf einer Seite der Kammer 61 befindet sich ein Gaseinlass 77, über den Moleküle in die Kammer 61 eingespeist werden können. Darüber hinaus weist die ECR-Ionenquelle eine Vorrichtung 79 zur Erzeugung von Mikrowellenstrahlung auf. Mithilfe dieser Mikrowellenstrahlung können bei geeigneter, resonanter Abstimmung der Frequenz der Mikrowellenstrahlung die Plasmaelektronen beschleunigt und damit das Plasma 63 geheizt werden. Die hierdurch entstehenden Ionen treten aus der ECR-Ionenquelle in einem Ionenstrom 81 aus.

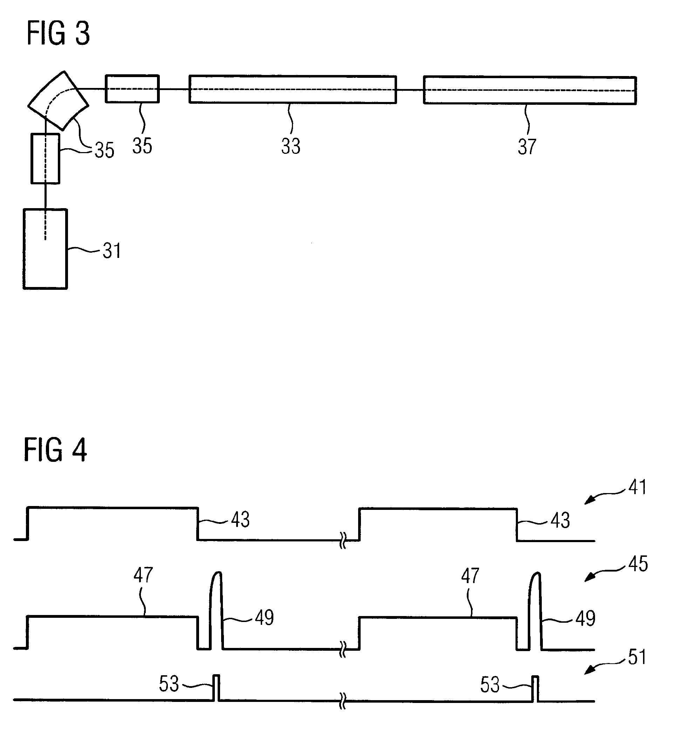

Fig. 3 zeigt einen schematischen Überblick über die ECR-Ionenquelle und die nachfolgende Beschleunigereinheit.

Anschließend an die ECR-Ionenquelle 31 folgt ein RFQ-Beschleuniger 33, der Ionenpulse, die in den RFQ-Beschleuniger 33 eintreten, beschleunigt. Dem RFQ-Beschleuniger 33 kann ein IH-Driftrohr-Linearbeschleuniger 37 folgen. Da die ECR-Ionenquelle 31 wie später anhand von Fig. 4 beschrieben im Afterglow-Modus betrieben ist, folgt der ECR-Ionenquelle 31 der RFQ-Beschleuniger 33 derart, dass kein Element zwischen ECR-Ionenquelle 31 und RFQ-Beschleuniger 33 angeordnet ist, das den aus der ECR-Ionenquelle 31 austretenden Ionenstrom "zerhackt", wie beispielsweise ein sonst üblicher Makropuls-Chopper. Zwischen der ECR-Ionenquelle 31 und dem RFQ-Beschleuniger 33 werden jedoch üblicherweise weitere strahlformende oder strahlmessende Elemente 35 wie fokussierende und/oder defokussierende Magnete, z.B. Solenoide, Quadrupolmagnete, Spektrometer-Magnete oder Strahldiagnosevorrichtungen angeordnet.

Nach der in Fig. 3 dargestellten linearen Beschleunigereinheit folgt üblicherweise eine weitere Beschleunigereinheit, wie beispielsweise ein Synchrotron.

Fig. 4 zeigt anhand von schematischen Diagrammen die zeitliche Abstimmung des Timings zwischen der ECR-Ionenquelle und dem nachfolgenden RFQ-Beschleuniger.

In der ersten Zeile 41 von Fig. 4 ist das Ein- und Ausschalten der Mikrowellenstrahlung in der ECR-Ionenquelle 31 dargestellt. Die Mikrowellenstrahlung wird dabei gepulst angewendet. Dies bedeutet, dass Mikrowellenstrahlung beispielsweise als ein Mikrowellen-Strahlungspuls 43 von 10 ms Dauer angewendet und anschließend pausiert wird. Wenn die ECR-Ionenquelle 31 mit einer Frequenz von z.B. 5 Hz betrieben wird, erfolgt die Applikation der 10 ms dauernden Mikrowellen-Strahlungspulse 43 im Abstand von 200 ms.

Solange ein Mikrowellen-Strahlungspuls 43 appliziert wird, wird ein dem Mikrowellen-Strahlungspuls entsprechender Ionenstrom aus der ECR-Ionenquelle 31 emittiert - hier in der zweiten Zeile 45 idealisiert mit einem rechteckigen Ionenstrom-Pulsverlauf 47 dargestellt. Nach Beenden des Mikrowellen-Strahlungspulses 43 wird aus der ECR-Ionenquelle 31 im Anschluss daran ein kurz dauernder Ionenstrom-Puls mit hoher Strahlintensität emittiert. Dieser in der "Nachglüh"-Phase emittierte Strahlenpuls wird auch als Afterglow-Strahlen-Puls 49 bezeichnet. Er zeichnet sich durch seine kurze Dauer von maximal wenigen Millisekunden und durch seine hohe Stromstärke - verglichen mit der Stromstärke des Ionenstroms während des Mikrowellen-Strahlungspulses 43 - aus.

In der dritten Zeile 51 ist schematisch das Timing des RFQ-Beschleunigers 33 dargestellt. Der RFQ-Beschleuniger 33 ist dabei zeitlich derart abgestimmt, dass sein HF-Puls 53 (z.B. mit einer üblichen Pulslänge von 300 ps) genau in die Phase des Afterglow-Strahlen-Pulses 49 fällt. Auf diese Weise werden diejenigen Ionen im RFQ-Beschleuniger 33 auf höhere Energien beschleunigt, die durch den Afterglow-Strahlen-Puls 49 bereitgestellt worden sind.

## Patentansprüche

1. Partikeltherapieanlage
mit einer ECR-Ionenquelle (31) zur Erzeugung von geladenen Ionen, die in einer der ECR-Ionenquelle (31) nachfolgenden Beschleunigereinheit (13, 15, 33, 37) auf eine zur Bestrahlung notwendige Energie beschleunigt werden,
wobei die Magnetfelder der ECR-Ionenquelle (31) auf einen Betrieb der ECR-Ionenquelle (31) für leichte Ionen abgestimmt sind, **dadurch gekennzeichnet, dass** die ECR-Ionenquelle (31) im Afterglow-Modus betreibbar ist,
bei dem nach Abschalten eines Mikrowellen-Resonanzpulses (43) ein Afterglow-Strahlen-Puls (49) aus der ECR-Ionenquelle (31) austritt, der eine höhere Stromstärke aufweist als ein Strom (47), der aus der ECR-Ionenquelle (31) während der Anwendung des Mikrowellen-Resonanzpulses (43) austritt.

2. Partikeltherapieanlage nach Anspruch 1, wobei die ECR-Ionenquelle (31) zum Betrieb mit Kohlenstoffionen und/oder Sauerstoffionen ausgebildet ist.

3. Partikeltherapieanlage nach Anspruch 1 oder 2, wobei die nachfolgende Beschleunigereinheit (13, 15, 33, 37) einen Linearbeschleuniger, insbesondere einen RFQ-Beschleuniger (33), umfasst, dessen Timing derart auf die im Afterglow-Modus betriebene ECR-Ionenquelle (31) abgestimmt ist, dass zumindest ein Teil des Ionenstroms, der während des Afterglow-Strahlen-Pulses (49) aus der ECR-Ionenquelle (31) austritt, durch den nachfolgenden Linearbeschleuniger beschleunigt wird.

4. Partikeltherapieanlage nach Anspruch 3, wobei der Linearbeschleuniger nach der ECR-Ionenquelle (31) folgt, ohne dass zwischen der ECR-Ionenquelle (31) und dem Linearbeschleuniger ein Strahlpuls formendes Element angeordnet ist.

5. Partikeltherapieanlage nach einem der Ansprüche 1 bis 4, wobei
die ECR-Ionenquelle (31) ein erstes Magnetsystem (71) zur Erzeugung eines axialen Magnetfeldes aufweist, das zumindest einen Permanentmagneten (73) umfasst und zumindest einen zusätzlich zum Permanentmagneten (73) variabel einstellbaren Elektromagneten (75).

6. Partikeltherapieanlage nach einem der Ansprüche 1 bis 5, wobei
die ECR-Ionenquelle (31) ein zweites Magnetsystem (65) zur Erzeugung eines radialen Magnetfeldes aufweist, das zumindest einen Permanentmagneten (69) umfasst und zumindest einen zusätzlich zum Permanentmagneten (69) variabel einstellbaren Elektromagneten (67).

## Claims

1. Particle therapy installation
having an ECR ion source (31) for the production of charged ions which are accelerated in an accelerator unit (13, 15, 33, 37) that is arranged downstream of the ECR ion source (31) to an energy required for irradiation, wherein the magnetic fields of the ECR ion source (31) are attuned to the operation of the ECR ion source (31) for lightweight ions, **characterised in that** the ECR ion source (31) can be operated in the afterglow mode, in which, after a microwave resonance pulse (43) has been switched off, an afterglow beam pulse (49) is emitted from the ECR ion source (31), the current intensity of which is higher than a current (47) which is emitted from the ECR ion source (31) during the application of the microwave resonance pulse (43).

2. Particle therapy installation according to claim 1, wherein the ECR ion source (31) is designed to operate with carbon ions and/or oxygen ions.

3. Particle therapy installation according to claim 1 or claim 2, wherein the accelerator unit that is arranged downstream (13, 15, 33, 37) encompasses a linear accelerator, in particular an RFQ accelerator (33), the timing of which is attuned to the ECR ion source (31) that is operated in afterglow mode in such a way that at least part of the ion current emitted from the ECR ion source (31) during the afterglow beam pulse (49) is accelerated by the linear accelerator that is arranged downstream.

4. Particle therapy installation according to claim 3, wherein the linear accelerator is arranged downstream of the ECR ion source (31) without an element that forms a beam pulse being arranged between the ECR ion source (31) and said linear accelerator.

5. Particle therapy installation according to one of claims 1 to 4, wherein
the ECR ion source (31) has a first system of magnets (71) for generating an axial magnetic field, which system encompasses at least one permanent magnet (73) and at least one variably adjustable electromagnet (75) in addition to the permanent magnet (73).

6. Particle therapy installation according to one of claims 1 to 5, wherein
the ECR ion source (31) has a second system of magnets (65) for the generation of a radial magnetic field, which system encompasses at least one permanent magnet (69) and at least one variably adjustable electromagnet (67) in addition to the permanent magnet (69).

## Revendications

1. Installation de thérapie par particules,
comportant une source d'ions ECR (31) destinée à générer des ions chargés lesquels sont accélérés, dans une unité d'accélérateur (13, 15, 33, 37) placée à la suite de la source d'ions ECR (31), pour atteindre une énergie nécessaire à l'irradiation,
les champs magnétiques de la source d'ions ECR (31) étant adaptés à un fonctionnement de la source d'ions ECR (31) pour ions légers,
**caractérisée en ce que** la source d'ions ECR (31) peut fonctionner en mode afterglow dans lequel, après coupure d'une impulsion de résonance à hyperfréquence (43), la source d'ions ECR (31) émet une impulsion de faisceau afterglow (49) qui présente une intensité du courant plus élevée qu'un courant (47) qui sort de la source d'ions ECR (31) pendant l'application de l'impulsion de résonance à hyperfréquence (43).

2. Installation de thérapie par particules selon la revendication 1,
la source d'ions ECR (31) étant conçue pour fonctionner avec des ions carbone et/ou des ions oxygène.

3. Installation de thérapie par particules selon la revendication 1 ou 2,
l'unité d'accélérateur (13, 15, 33, 37) placée en aval comprenant un accélérateur linéaire, en particulier un accélérateur RFQ (33), dont la synchronisation est adaptée à la source d'ions ECR (31) fonctionnant en mode afterglow, de telle sorte qu'au moins une partie du courant ionique sortant de la source d'ions ECR (31) pendant l'impulsion de faisceau afterglow (49) est accélérée par l'accélérateur linéaire placé à la suite.

4. Installation de thérapie par particules selon la revendication 3,
l'accélérateur linéaire étant placé à la suite de la source d'ions ECR (31) sans qu'un élément formant une impulsion de faisceau soit intercalé entre la source d'ions ECR (31) et l'accélérateur linéaire.

5. Installation de thérapie par particules selon l'une des revendications 1 à 4,
la source d'ions ECR (31) comportant un premier système magnétique (71) destiné à produire un champ magnétique axial, qui comprend au moins un aimant permanent (73) et au moins un électroaimant (75) pouvant être réglé de manière variable en plus de l'aimant permanent (73).

6. Installation de thérapie par particules selon l'une des revendications 1 à 5,
la source d'ions ECR (31) comportant un second système magnétique (65) destiné à produire un champ magnétique radial, qui comprend au moins un aimant permanent (69) et au moins un électroaimant (67) pouvant être réglé de manière variable en plus de l'aimant permanent (69).
